# EUROPÄISCHE PATENTSCHRIFT

(11) **EP 1 089 824 B1**
(45) Veröffentlichungstag und Bekanntmachung des Hinweises auf die Patenterteilung: **05.11.2003**
(21) Anmeldenummer: 99934541.6
(22) Anmeldetag: 28.06.1999
(51) Int. Cl.: B03C 5/02

(54) **ELEKTRODENANORDNUNG ZUR DIELEKTROPHORETISCHEN PARTIKELABLENKUNG**
ELECTRODE ARRANGEMENT FOR THE DIELECTROPHORETIC DIVERSION OF PARTICLES
DISPOSITIF A ELECTRODES DESTINE A LA DEVIATION ELECTROPHORETIQUE DE PARTICULES

(30) Priorität: 26.06.1998 DE 19828626; 29.06.1998 DE 19828919; 20.11.1998 DE 19853658; 23.12.1998 DE 19860117
(43) Veröffentlichungstag der Anmeldung: 11.04.2001
(73) Patentinhaber: Evotec OAI AG, 22525 Hamburg (DE)
(72) Erfinder: FUHR, Günter, D-13187 Berlin (DE); SCHNELLE, Thomas, D-10243 Berlin (DE); HAGEDORN, Rolf, D-13057 Berlin (DE); MÜLLER, Torsten, D-12439 Berlin (DE)
(74) Vertreter: Hertz, Oliver, Dr.
(86) Internationale Anmeldenummer: EP9904469
(87) Internationale Veröffentlichungsnummer: WO00000292

(56) Entgegenhaltungen:
- US-A- 4 134 744
- US-A- 4 726 904
- US-A- 5 565 105
- FIEDLER S ET AL: "DIELECTROPHORETIC SORTING OF PARTICLES AND CELLS IN A MICROSYSTEM" ANALYTICAL CHEMISTRY, Bd. 70, Nr. 9, 1. Mai 1998 (1998-05-01), Seiten 1909-1915, XP000755524 ISSN: 0003-2700
- M.MADOU ET AL: "LabCD:A Centrifuge-Based Microfluidic Platform for Diagnostics" SPIE, Bd. 3259, 26. - 27. Januar 1998, Seiten 80-93, XP002116851 US in der Anmeldung erwähnt

## Beschreibung

Die Erfindung betrifft Mikrosysteme, die zur Handhabung suspendierter Teilchen oder biologischer Zellen eingerichtet sind, insbesondere die Gestaltung von Elektroden zur dielektrophoretischen Ablenkung von Teilchen oder Zellen, und Anwendungen derartiger Mikrosysteme.

Es ist bekannt, in Mikrosystemen mit Elektroden-Kanal-Anordnungen flüssigkeitssupendierte Teilchen auf der Grundlage negativer oder positiver Dielektrophorese zu manipulieren, wobei unter der Wirkung hochfrequenter elektrischer Felder Polarisationskräfte erzeugt werden, die eine Abstoßung von den Elektroden und in Zusammenwirkung mit Strömungskräften in der Suspensionsflüssigkeit eine Manipulation der Teilchen im Mikrosystem erlauben. Eine Übersichtsdarstellung zu den bekannten Mikrosystemen wird z.B. von G. Fuhr et al. in "Naturwissenschaften", Band 81, 1994, Seite 528 ff., gegeben.

Herkömmliche Mikrosysteme besitzen Nachteile in Bezug auf die Stabilität und Lebensdauer der Elektroden und die Beschränkung auf bestimmte Potentialformen entsprechend der jeweiligen Elektrodengeometrie.

So besitzen die Mikroelektroden herkömmlicher Mikrosysteme in der Regel die Form gerader Bänder, die zur Erzielung bestimmter Feldbarrieren im Kanal eines Mikrosystems in bestimmter Weise in Bezug auf den Kanal ausgerichtet sind. Aufgrund mechanischer Beanspruchung oder durch Materialermüdung oder auch durch Herstellungsfehler kann es zu Unterbrechungen in den geraden Elektrodenbändern und damit zum Funktionsausfall des gesamten Mikrosystems kommen. Ein herkömmliches System ist ferner entsprechend der gegebenen Elektrodenstrukturierung auf eine bestimmte Funktion beschränkt. Variable Wirkungen zur Teilchenablenkung in einem gegebenen Mikrosystem sind nicht möglich.

Aus der Publikation von S. Fiedler et al. in Anal. Chem. "Band 70, 1998, Seite 1909 ff. ist ein Mikrosytem zum dielektrophoretischen Sortieren von Partikeln und Zellen bekannt. Das Mikrosystem enthält eine Elektrodenanordnung mit einer Vielzahl von streifenförmigen, dreieckigen oder rechteckigen Elektroden.

Die Aufgabe der Erfindung ist es, verbesserte Elektrodenanordnungen für Mikrosysteme mit dielektrophoretischer Partikelablenkung zu schaffen, mit denen die Nachteile herkömmlicher Mikrosysteme überwunden werden. Die Aufgabe der Erfindung ist es ferner, Anwendungen derartiger Elektrodenanordnungen anzugeben.

Diese Aufgabe wird durch ein Mikrosystem mit den Merkmalen gemäß dem Patentanspruch 1 gelöst. Vorteilhafte Ausführungsformen und Anwendungen der Erfindung ergeben sich aus den abhängigen Ansprüchen.

Die Grundidee der Erfindung besteht insbesondere darin, bei einer Elektrodenanordnung in einem Mikrosystem mit dieelektrophoretischer Partikelablenkung, die aus mindestens einer Elektrode besteht, eine Unterteilung der Elektrode in Elektrodensegmente vorzusehen. Die Elektrodensegmente sind dazu eingerichtet, gemeinsam oder separat mit Potentialen beaufschlagt zu werden und gemeinsam zusammenwirkend eine Feldbarriere entsprechend der Funktion der jeweiligen Elektrode im Mikrosystem zu bilden. Die Elektrodensegmente sind gegenüber einer Flüssigkeit im Mikrosystem freiliegende Elektrodenflächen, die je nach Gestaltungsform miteinander elektrisch verbunden, wobei dann die Bereiche der elektrischen Verbindungen gegenüber der Flüssigkeit im Mikrosystem nicht freiliegen, d.h. abgedeckt sind, oder elektrisch voneinander isoliert sind. Der Übergang von herkömmlichen, flächigen oder bandförmigen Elektroden zur erfindungsgemäßen Elektrodensegmentierung löst die oben genannte Aufgabe in vorteilhafter Weise in mehrfacher Hinsicht. Einerseits sind die Elektrodensegmente weniger störanfällig, wie dies im einzelnen unten erläutert wird. Andererseits erlauben sie auch bei separater Ansteuerbarkeit eine in herkömmlichen Mikrosystemen nicht gegebene Multifunktionalität der Mikroelektroden und damit der Mikrosysteme an sich.

Erfindungsgemäß werden die Elektrodensegmente dadurch gebildet, dass Elektroden mit an sich flächiger oder bandförmiger Ausdehnung eine Isolationsschicht tragen, die in vorbestimmten Teilen Ausnehmungen besitzt. Die Ausnehmungen weisen die Gestalt und Position der gewünschten Elektrodensegmente auf. Durch die Ausnehmungen tritt die Flüssigkeit im Mikrosystem in Kontakt mit der Elektrode, die wegen der isolierenden oder dielektrischen Abdeckung lediglich durch die Ausnehmungen bzw. Elektrodensegmente wirksam und im übrigen unwirksam ist. Diese Gestaltung ist vorteilhaft für die Lebensdauer der Elektroden, da selbst eine Durchtrennung des gesamten Elektrodenteils, das zur Flüssigkeit hin offen liegt, nicht zum Ausfall der Elektrode führt.

Gemäß einer bevorzugten Ausführungsform der Erfindung sind die Elektrodensegmente einzeln, unabhängig voneinander ansteuerbar. Die gemeinsam eine Elektrodenfunktion übernehmenden Elektrodensegmente sind im Mikrosystem z.B. an einer Kanalwand in einem Bereich angeordnet, dessen Form einer herkömmlichen Elektrode entspricht, die zur Erfüllung dieser Funktion vorgesehen wäre. Die Elektrodensegmente sind separat mit Potentialen beaufschlagbar, die anwendungsabhängig in Bezug auf die Phasenlage und Amplituden variieren.

Gemäß einer weiteren Ausführungsform der Erfindung sind Elektrodensegmente, die voneinander elektrisch isoliert und einzeln ansteuerbar sind, als Elektrodenarray angeordnet. Ein Elektrodenarray besteht aus einer Vielzahl punkt- oder flächenförmiger Elektrodensegmente, die beispielsweise matrixartig in Reihen und Spalten oder anwendungsabhängig in anderen geomerischen Konfigurationen angeordnet sind und von denen bei Betrieb des Mikrosystems eine vorbestimmte Anzahl von Elektrodensegmenten zur Erzeugung einer bestimmten Elektrodenfunktion mit elektrischen Potentialen beaufschlagt sind, während die übrigen Elektrodensegmente des Elektrodenarrays nicht angesteuert werden. Dies ist eine besonders vorteilhafte Gestaltung einer erfindungsgemäßen Elektrodenanordnung, da je nach Anwendung die Elektrodensegmente verschieden angesteuert und somit die Elektrodenfunktion frei gewählt werden kann. Diese Funktionswahl kann, wie unten erläutert wird, irreversibel oder reversibel erfolgen.

Weitere wichtige Gesichtspunkte der Erfindung sind die geometrische Konfiguration von Elektrodensegmenten, mit denen Gradienten und somit verschieden starke Kräfte erzeugt werden können und/oder die an das Strömungsprofil in der Suspensionsflüssigkeit angepaßt sind. Letztere Gestaltung besitzt den Vorteil, daß die Elektroden kürzer ausgebildet werden können und mit geringeren Kräften behaftet sind, jedoch die gleiche Effektivität wie herkömmliche Mikroelektroden besitzen.

Bevorzugte Anwendungen der Erfindung liegen in der Technik fluidischer Mikrosysteme zur Separation, Manipulation, Beladung, Fusion, Permeation, Pärchenbildung und Aggregatformation von mikroskopisch kleinen, suspendierten Partikeln (synthetische Teilchen und/oder biologische Teilchen, wie z.B. biologische Zellen, Zellbestandteile oder Makromoleküle).

Gemäß einer besonderen Ausführungsform der Erfindung erfolgt die Teilchenbewegung in einem Mikrosystem mit herkömmlichen oder erfindungsgemäßen Elektrodenformen unter der Wirkung von Zentrifugal- und/oder Gravitationskräfen.

Weitere Einzelheiten und Vorteile der Erfindung werden aus den im folgenden beschriebenen Zeichnungen ersichtlich. Es zeigen:
- Fig. 1: eine schematische Perspektivansicht einer Kanalstruktur mit Mikroelektroden zur Erzeugung von Kraftbarrieren in einem Mikrokanal,
- Fig. 2: eine erste Ausführungsform der Erfindung mit bandartigen Elektrodensegmenten,
- Fig. 3: weitere Ausführungsformen der Erfindung mit punkt- oder streifenförmigen Elektrodensegmenten,
- Fig. 4: weitere Ausführungsformen mit bandförmig angeordneten Elektrodensegmenten zur Erzeugung von Feldgradienten, wobei Fig. 4b keine erfindungsgemäße Ausführungsform darstellt,
- Fig. 5: eine schematische Draufsicht auf einen Mikrokanal mit bandförmigen Elektrodensegmenten zur Bildung eines Partikeltrichters,
- Fig. 6: eine schematische Draufsicht auf einen weiteren Partikeltrichter aus Elektrodensegmenten,
- Fig. 7: eine schematische Draufsicht auf einen weiteren gegenüber Fig. 6 abgewandelten Partikeltrichter,
- Fig. 8: eine schematische Draufsicht auf ein Elektrodenarray in einem erfindungsgemäßen Mikrosystem,
- Fig. 9: eine Illustration eines Ansteuerbeispiels für ein Elektrodenarray gemäß Fig. 7,
- Fig. 10: eine Illustration eines weiteren Ansteuerbeispiels eines Elektrodenarrays gemäß Fig. 7,
- Fig. 11: weitere Ausführungsformen von Elektrodensegmenten mit Schleifen und Mehrfacheinspeisungen,
- Fig. 12: eine weitere Ausführungsform einer programmierbaren Elektrodenanordnung, und
- Fign. 13 bis 15: Beispiel eines Mikrosystems mit Zentrifugal- und/oder Gravitationsantrieb der Teilchenbewegung.

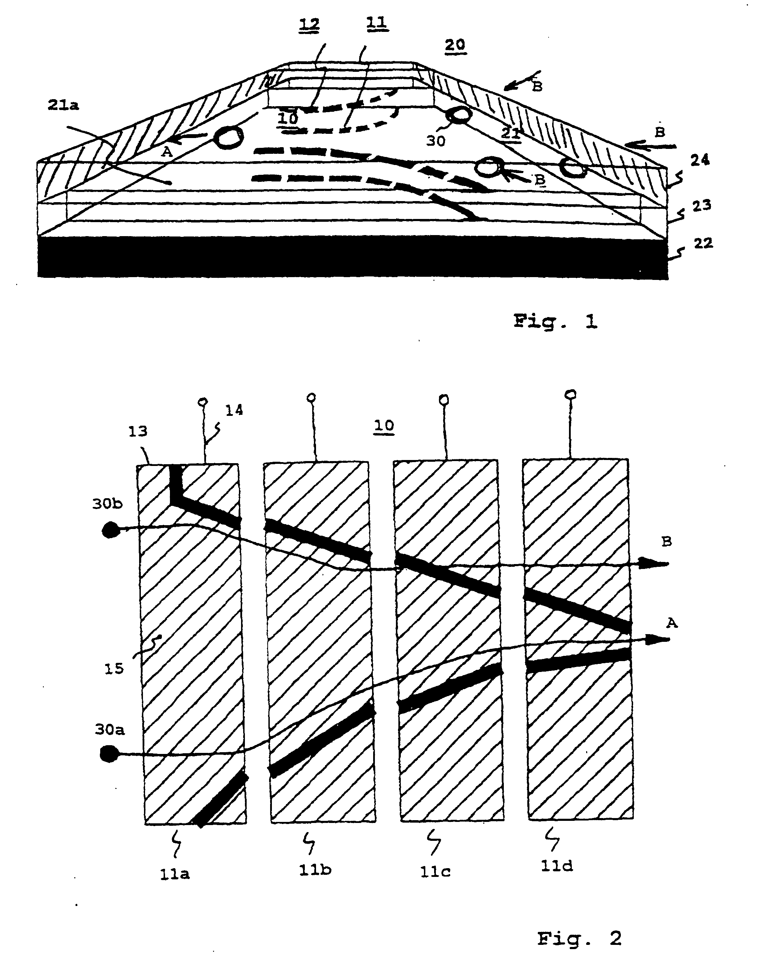

Fig. 1 zeigt in schematischer Form beispielhaft die Ausführung von Mikroelektroden zur Erzeugung von Kraftbarrieren in Mikrokanälen. Das fluidische Mikrosystem 20 ist ausschnittsweise in überhöht perspektivischer Seitenansicht einer Kanalstruktur dargestellt. Der Kanal 21 wird beispielsweise durch zwei mit Abstand auf einem Substrat 22 angeordnete Spacer 23 gebildet, die ein Deckteil 24 tragen. Derartige Strukturen werden beispielsweise mit den an sich bekannten Prozessierungstechniken der Halbleitertechnologie hergestellt. Das Substrat 22 bildet die Bodenfläche 21a des Kanals 21. Dementsprechend wird die Deckfläche 21b (aus Übersichtlichkeitsgründen nicht gesondert hervorgehoben) durch das Deckteil 24 gebildet. Die Elektrodenanordnung 10 besteht aus Mikroelektroden 11, 12, die auf der Bodenfläche 21a bzw. auf der Deckfläche 21b angebracht sind. Jede der Mikroelektroden 11, 12 besteht aus mehreren Elektrodensegmenten, die unten näher beschrieben werden.

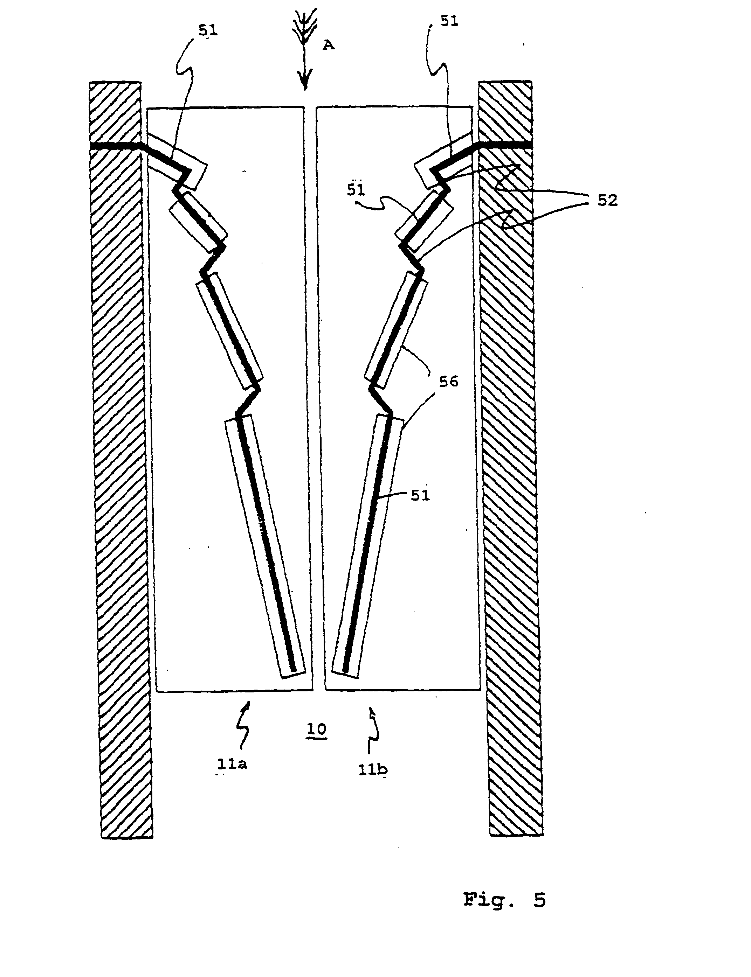

In Fig. 1 bilden die Elektrodensegmente eine Elektrodenstruktur, die im einzelnen unten unter Bezug auf die Fign. 5-7 erläutert wird. Die anderen, im folgenden beschriebenen Ausführungsformen erfindungsgemäßer Elektrodenanordnungen können entsprechend auf den Boden- und/oder Deckflächen des Kanals 21 angebracht sein. Der Mikrokanal 21 wird von einer Suspensionsflüssigkeit durchströmt (im Bild von rechts nach links), in der Partikel 30 suspendiert sind. Die in Fig. 1 dargestellte Elektrodenanordnung 10 besitzt beispielsweise die Aufgabe, die Partikel 30 von verschiedenen Bewegungsbahnen innerhalb des Kanals auf eine mittlere Bewegungsbahn gemäß Pfeil A zu führen. Hierzu werden die Mikroelektroden 11, 12 derart mit elektrischen Potentialen beaufschlagt, daß sich im Kanal elektrische Feldbarrieren ausbilden, die die von rechts anströmenden Teilchen hin zur Kanalmitte (Pfeilrichtungen B) zwingen.

Die typischen Abmessungen der Mikroelektroden 11, 12 liegen bei einer Breite von 0,1 bis zu einigen zehn Mikrometern (typischerweise 5 ... 10 µm), einer Dicke von 100 nm bis zu einigen Mikrometern (typischerweise 200 nm) und einer Länge von bis zu mehreren hundert Mikrometern. Die Länge der Elektrodensegmente ist anwendungsabhängig in Abhängigkeit von ihrer Zahl und ihrem jeweiligen Abstand entsprechend kürzer. Das Innere des Kanals 21 wird durch die auf der Ober- und Unterseite der Teile 23, 24 prozessierten Elektroden auf Grund der geringfügigen Dicke der Elektroden nicht eingeschränkt. Das Teil 23 ist ein Spacer, dessen Strukturierung die seitlichen Kanalwände bildet.

Die Mikroelektroden 11, 12 werden mittels hochfrequenter elektrischer Signale (typischerweise mit einer Frequenz im MHz-Bereich und einer Amplitude im Voltbereich) angesteuert. Die jeweils gegenüberliegenden Elektroden 11a, 11b bilden ein Ansteuerpaar, wenngleich auch die in einer Ebene liegenden Elektroden in ihrer Ansteuerung (Phase, Frequenz, Amplitude) zusammenwirken. Das durch den Kanal 21, d.h. senkrecht zur Strömungsrichtung erzeugte elektrische Hochfrequenzfeld wirkt auf suspendierte Teilchen 30 (die auch lebende Zellen oder Viren sein können) polarisierend. Bei den genannten Frequenzen und geeigneter Leitfähigkeit der die Teilchen umgebenden Suspensionsflüssigkeit werden die Teilchen von den Elektroden abgestoßen. Damit läßt sich der hydrodynamisch offene Kanal 21 über die elektrischen Felder an- und abschaltbar strukturieren, kompartimentieren bzw. lassen sich die Bewegungsbahnen der Teilchen im passiven Strömungsfeld beeinflussen. Desweiteren ist es möglich, die Teilchen trotz permanenter Strömung zu retardieren bzw. auch ortsstabil ohne Berührung einer Oberfläche zu positionieren.

Im folgenden werden Gestaltungsformen erfindungsgemäßer Elektrodenanordnungen beschrieben, wobei aus Übersichtlichkeitsgründen in den Figuren jeweils nur eine planare Elektrodenanordnung (oder Teile einer solchen), z.B. auf der Bodenfläche des Kanals, dargestellt ist.

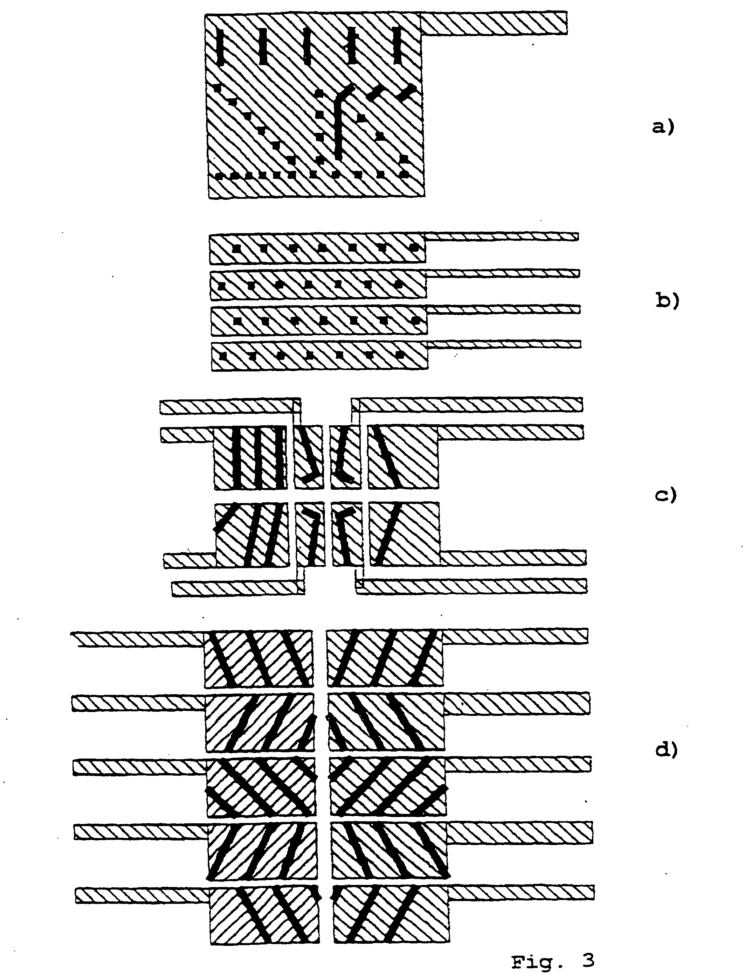

Für die Erzeugung von elektromagnetischen Begrenzungen in Kanalsystemen von Mikrostrukturen sind schmale, bandartige Elektroden verschiedener Geometrie günstig, da die Verluste proportional zur wirksamen Elektrodenfläche zunehmen. Derart schmale Elektroden sind jedoch gegenüber Produktionsfehlern und lokalen Unterbrechungen sehr empfindlich. So führt ein Haarriß bereits zum Ausfall des gesamten restlichen Teils einer Bandelektrode. Bei der in Fig. 2 dargestellten Ausführungsform werden schmale Bandelektroden ohne die genannten Nachteile realisiert.

Die Elektrodenanordnung 10 besteht aus vier separat ansteuerbaren Einzelelektroden 11a-11d. Jede Einzelelektrode wird durch eine rechteckige metallische Beschichtung 13 z.B. auf der Bodenfläche des Kanals mit einer zugehörigen Steuerleitung 14 gebildet. Die Schichtdicke liegt im Bereich von 50 nm bis zu einigen Mikrometern und beträgt vorzugsweise rd. 200 nm. Die Metallschicht 13 trägt eine strukturierte Isolationsschicht 15 (schraffiert dargestellt). Die Isolationsschicht 15 ist derart strukturiert, daß entlang bestimmter Ausnehmungen die Metallschicht 13 freiliegt (schwarz dargestellt). Die freiliegenden Bereiche bilden die Elektrodensegmente, an denen die Suspensionsflüssigkeit im Kanal direkt mit der Elektrode in Kontakt kommt. Falls im Bereich eines Elektrodensegments etwa ein Haarriß oder ein anderweitiger Fehler auftritt, so wird über die übrige Metallschicht sichergestellt, daß alle Teile des Elektrodensegments dennoch mit den gewünschten elektrischen Potentialen beaufschlagt werden.

Bei der Ausführungsform gemäß Fig. 2 sind die nebeneinander angeordneten Einzelelektroden 11a-11d so strukturiert, daß zwei Reihen von Elektrodensegmenten gebildet werden. Die Elektrodensegmente jeweils einer Reihe, die gerade oder gekrümmt sein kann, wirken zur Bildung vorbestimmter Feldbarrieren analog zur Funktion einer herkömmlichen Mikroelektrode zusammen. Je nach Ansteuerung der Einzelelektroden können dabei beispielsweise die folgenden Funktionen erzielt werden.

Werden die Einzelelektroden zur Ausbildung einer in Kanalrichtung trichterförmigen Feldbarriere (Partikeltrichter) angesteuert, so werden sämtliche Partikel 30a, 30b hin zur Kanalmitte geführt, wie dies oben erläutert wurde. Alternativ ist es aber auch möglich, eine oder mehrere der Einzelelektroden zeitweilig abzuschalten, so daß einzelne Partikel 30a hin zur Kanalmitte geführt werden (Pfeil A), während andere Partikel 30b mit Abstand von der Kanalmitte weiter strömen. Beim dargestellten Beispiel wurde die Einzelelektrode 11c unmittelbar vor Erreichen durch den Partikel 30b kurzzeitig abgeschaltet, so daß in diesem Bereich die Feldbarriere im Kanal wegfiel. Dadurch kann der Partikel 30b entsprechend Pfeil B weiter bewegt werden. Für die Erzielung der Bahn des Partikels 30a sind sämtliche Einzelelektroden dauernd eingeschaltet.

Die Isolationsschichten bestehen bei sämtlichen Ausführungsformen vorzugsweise aus biokompatiblen Materialien, z.B. Oxiden SiO₂, SiNO₃, und dergleichen), Polymeren, Tantanverbindungen oder dergleichen. Es können auch aufgesputterte Materialien, die elektrisch isolierend sind, verwendet werden. Die Dicke der Isolationsschicht liegt im Bereich oberhalb 100 nm und kann bis zu einigen Mikrometer betragen.

Fig. 3 zeigt beispielhaft weitere Ausführungen a, b, c, d punktförmiger und streifenförmiger Elektrodensegmente sowie segmentierter Elektroden analog zu der in Fig. 2 erläuterten Elektrodenausführung. Die schraffierten Flächen stellen jeweils die mit einer Isolationsschicht bedeckten Metallschichten der Einzelelektroden dar, während die schwarz gefüllten Streifen bzw. Punkte die Elektrodensegmente zeigen. Die Elektrodensegmente sind im Kanal anwendungsabhängig angeordnet. Die Überströmung der Elektroden erfolgt jeweils in der Bildebene von oben nach unten (oder umgekehrt). Der Vorteil der separierten Elektrodenausführung besteht darin, daß durch die externe Ansteuerung der wirksame Verlauf der Bandelektroden hinsichtlich der Teilchenbewegung in weiten Bereich frei variiert werden kann.

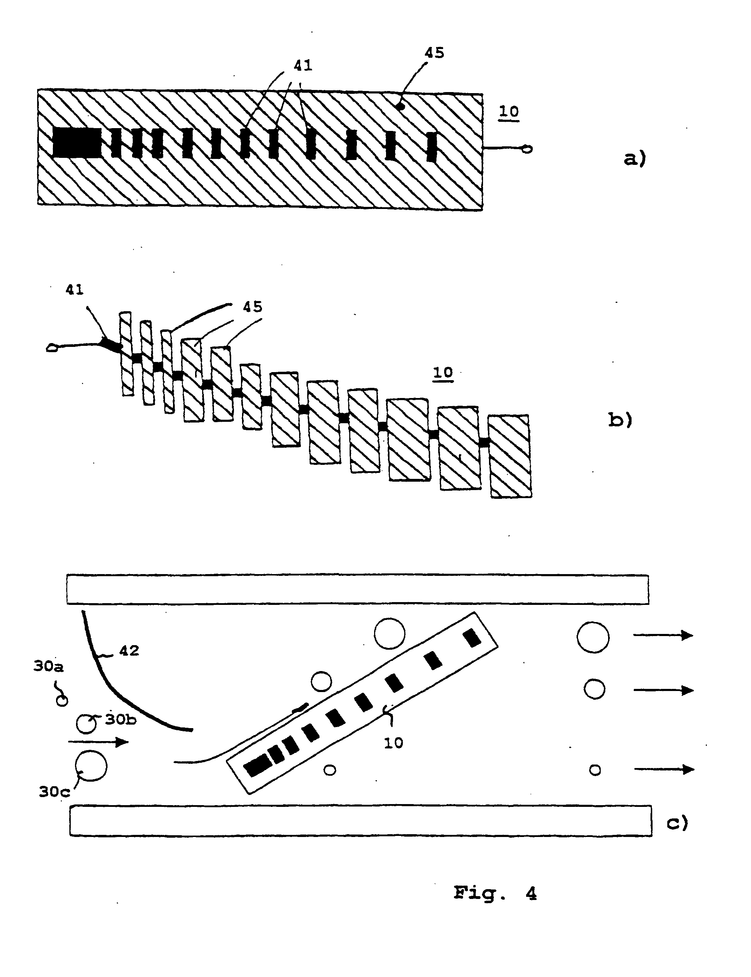

Für das Sortieren von Teilchen oder Zellen (z.B. nach dielektrischen Eigenschaften oder der Größe) ist es erforderlich, die Feldstärke über die Länge eines Elektrodenbandes zu variieren. Zwei mögliche Ausführungen sind in den Fign. 4a, 4b dargestellt. In der Ausführung a wird durch die Abstände der Ausnehmungen bzw. Elektrodensegmente 41 in der Isolationsschicht 45 ein Feldgradient erzeugt. Gemäß Ausführung b läßt sich dies über das Aufbringen verschieden breiter Isolierflächen 45 auf eine Bandelektrode erreichen.

Die Elektrodenanordnungen 10 gemäß den Fign. 4a, 4b sind im Mikrosystem so angeordnet, daß der Feldgradient eine bestimmte Ausrichtung in Bezug auf die Strömungsrichtung im Kanal besitzt. Wird beispielsweise ein Feldgradient schräg zur Kanallängsrichtung ausgebildet, so bedeutet dies, daß die anströmenden Partikel auf eine Feldbarriere mit in Kanalquerrichtung veränderlicher Amplitude treffen. Kleine Teilchen, bei denen auch bei hohen Amplituden nur geringe Polarisationskräfte auftreten, können die Feldbarriere bei hohen Amplituden überwinden, wohingegen größere Teilchen durch die Feldbarriere in Kanalquerrichtung so weit abgelenkt werden, bis die Polarisationskräfte genügend gering sind und die Feldbarriere durchlaufen werden kann. Eine erfindungsgemäße Elektrodenanordnung, deren Elektrodensegmente Feldgradienten bilden, ist somit zur Partikelsortierung in Abhängigkeit von der Ausbildung von Polarisationskräften im jeweiligen Partikel und somit in der Regel in Abhängigkeit von dessen Größe einsetzbar.

Das Prinzip dieser Partikelsortierung ist in Fig. 4c illustriert. Kleine Partikel 30a können die Feldbarriere der Elektrodenanordnung 10 gemäß Fig. 4a bei großen Feldstärken durchdringen, während größere Partikel 30b, 30c erst bei geringeren Feldstärken in Kanalrichtung weitergeführt werden. Die hierzu erforderlichen Amplituden werden anwendungsabhängig je nach den auftretenden Strömungs- und Polarisationskräften gewählt. Dies kann unter Verwendung der an sich bekannten Steuerprinzipien aus der Mikrosystemtechnik, insbesondere aus der Manipulierung von Partikeln auf der Basis negativer Dielektrophorese erfolgen. Die Elektrode 42 dient der Zuführung der Partikel zum Beginn des Feldgradienten.

Die in Fig. 2 dargestellte Abdecktechnik zur Herstellung bandartiger Elektroden kann auch zu ihrer Optimierung gemäß Fig. 5 genutzt werden. Fig. 5 zeigt eine Abwandlung einer Elektrodenanordnung 10 zur Bildung einer trichterförmigen Feldbarriere. Die Elektrodenanordnung 10 besteht aus zwei Einzelelektroden 11a, 11b, die jeweils die Form gekrümmter Elektrodenbänder besitzen. Jede der Einzelelektroden 11a, 11b ist von einer Isolationsschicht 55 mit Ausnehmungen 56 abgedeckt. Die Ausnehmungen 56 lassen vorbestimmte Abschnitte der Einzelelektroden 11a bzw. 11b frei, die die Elektrodensegmente 51 bilden. Die Teile 52 der Einzelelektroden 11a bzw. 11b sind wegen der abdeckenden Isolationsschicht elektrisch nicht wirksam.

Die Elektrodenbänder der Einzelelektroden sind winkelig derart ausgeführt, daß sich immer ein Elektrodenabschnitt, der hin zur Kanalmitte führt und den Elektrodensegmenten 51 entspricht, von einem Elektrodenabschnitt begrenzt wird, der von der Kanalmitte weg weist und den abgedeckten Rückführungen 52 entspricht. Diese Anordnung ermöglicht ein nahtloses Zusammenwirken der Elekrodensegmente, die zwar geometrisch voneinander getrennt sind, sich in Strömungsrichtung (s. Pfeil A) jedoch überlappende Feldbarrieren bilden.

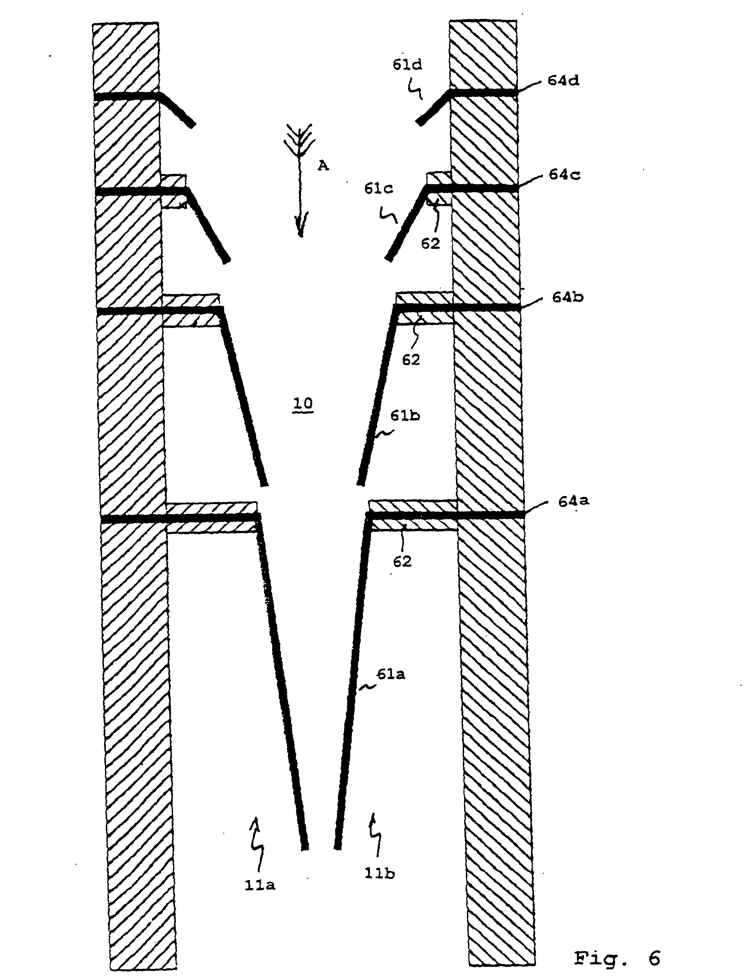

Am Beispiel des Partikeltrichters gemäß Fig. 6 soll noch eine weitere Elektrodenanordnung erläutert werden. In Medien hoher Luftfeuchtigkeit, wie sie z.B. für die Kultur tierischer und humaner Zellen verwendet werden (oder auch im Meerwasser), können die Verluste auf einer Bandleitung (bandförmige Elektrode) so groß sein, daß an deren Ende deutlich geringere oder gar keine Feldeffekte bezüglich der Partikelabdeckung mehr auftreten. Unter derartigen Umständen ist es zweckmäßig, die Elektroden 11a, 11b jeweils in Elektrodensegmente 61a bis d zu teilen und verschiedene Einspeisungen an den Steuerleitungen 64a bis d vorzunehmen. Die Winkel zum Kanalverlauf (Pfeil A) sind dem Strömungsprofil im Kanal angepaßt. Zuführende Teile 62 der Elektrodenanordnung 10 sind zweckmäßigerweise zu isolieren.

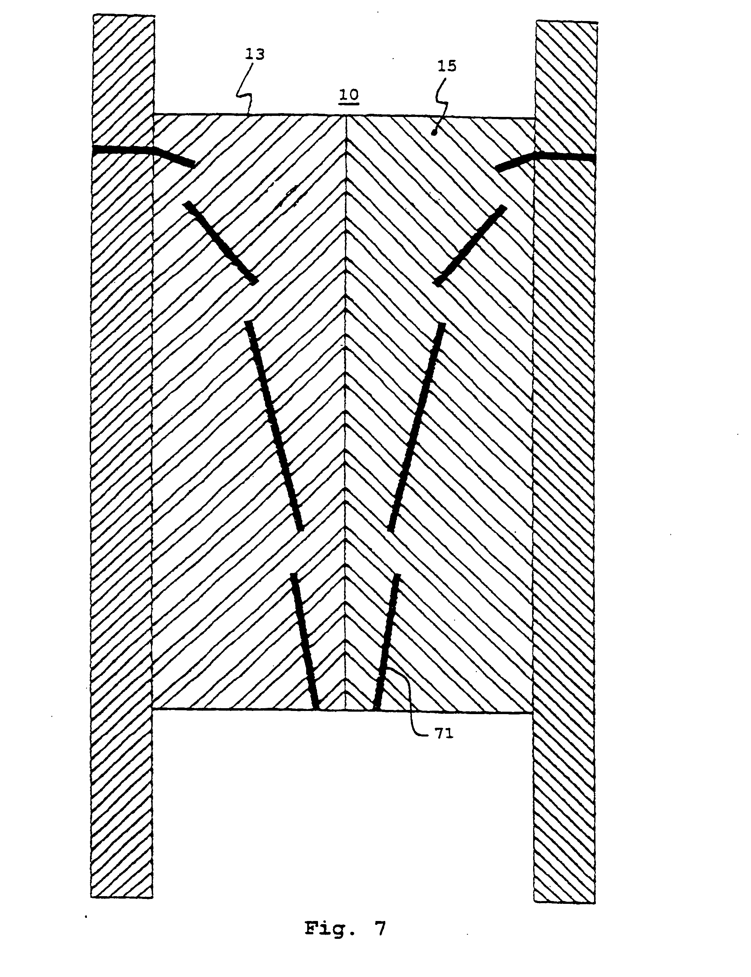

Alternativ zu der Darstellung gemäß Fig. 5 können die Einzelelektroden jeweils auch durch flächige Metallschichten nach dem in Fig. 2 erläuterten Prinzip gebildet werden. Die Elektroden können auch durch eine gemeinsame Metallschicht 13, die eine gemeinsame Isolationsschicht 15 mit Ausnehmungen entsprechend den gewünschten Elektrodensegmenten 71 trägt, gebildet werden (s. Fig. 7).

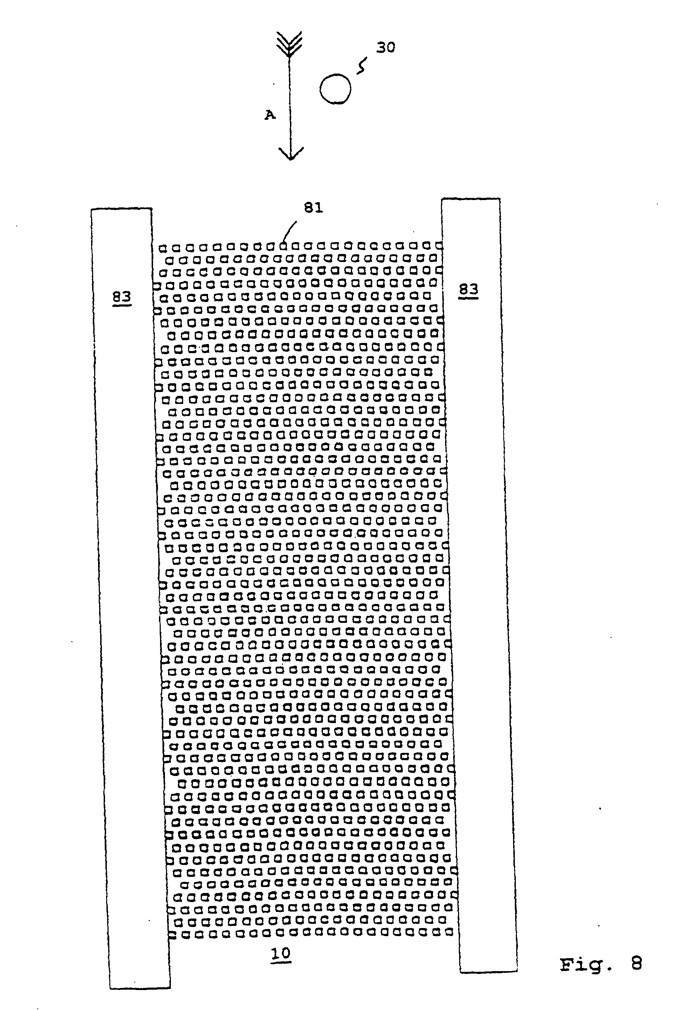

Im folgenden wird eine bevorzugte Ausführungsform der Erfindung unter Bezug auf die Fign. 8 bis 10 erläutert, bei der die Elektrodenanordnung 10 aus einer großen Anzahl von punktförmigen, matrixartig angeordneten Elektrodensegmenten 81 besteht, die sämtlich einzeln ansteuerbar sind.

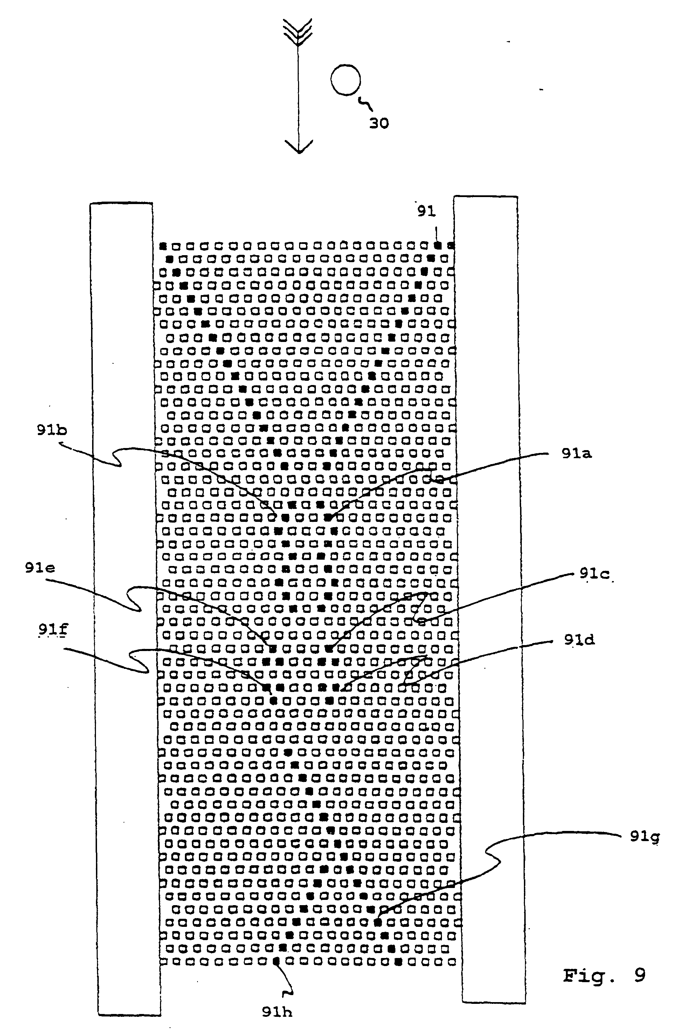

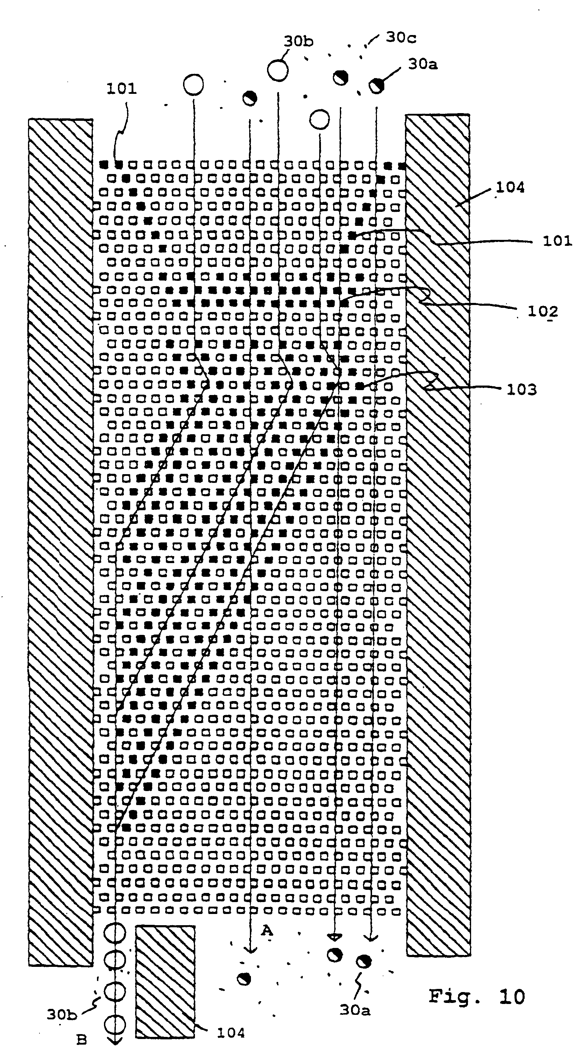

Fig. 8 zeigt beispielhaft die Anordnung von Elektrodensegmenten 81 auf der Boden- und/oder Deckfläche des Kanals. Die Elektrodenanordnung erstreckt sich vorzugsweise über die gesamte Kanalbreite zwischen den Spacern 83, die die seitlichen Kanalwände bilden. Ein oder mehrere Partikel 30 strömen beispielsweise in Pfeilrichtung (Pfeil A) über die Elektrodenanordnung 10. Mit den Elektrodensegmenten 81 lassen sich beliebige Wirkungen zur Partikelablenkung, insbesondere wie sie in Fig. 1 bis 7 dargestellt wurden, in programmierbarer Weise über die einzeln ansteuerbaren punktförmigen oder quadratischen bzw. rechteckigen Elektrodensegmente in arrayartiger Anordnung erreichen, wenn die Abstände zwischen den Elektrodensegmenten 81 kleiner als die zu manipulierenden Partikel 30 sind. Vorzugsweise ist eine gleichartige Elektrodenanordnung auf der Oberseite des Kanals angebracht, so daß sich elektrische Hochfrequenzfelder von der Oberseite des Kanals zur Unterseite des Kanals ausbilden können. Beispiele für eine mögliche Ansteuerung sind in Fig. 9 und 10 dargestellt.

Fig. 9 zeigt beispielhaft die Ansteuerung eines Elektrodenarrays, wie es in Fig. 8 erläutert wurde. Die hellen Elektrodensegmente sind nicht angesteuert. Die schwarz gezeichneten Elektrodensegmente 91, 91a-h werden mit einer Wechselspannung (z.B. zwischen 1 und 500 MHz) angesteuert. An den entsprechenden Positionen werden auch die in der oberen Ebene des Kanals (hier nicht dargestellt) liegenden Elektrodensegmente angesteuert. Als Beispiel wird die Ansteuerung der Elektrodensegmente im folgenden in Tabellenform aufgelistet. Dabei beschreiben die ungestrichenen Bezugszeichen Gruppen von Elektrodensegmenten der unteren Ebene, während Zahlen mit einem (') sich auf die obere Kanalebene beziehen:

| **Elektroden** | **Phasenlage** | **Elektrode** | **Phasenlage** |
|---|---|---|---|
| 91 | 0° | 91f | 270° |
| 91' | 180° | 91f' | 90° |
| 91a | 0° | 91g | 0° |
| 91a' | 180° | 91g' | 180° |
| 91b | 0° | 91h | 0° |
| 91b' | 180° | 91h' | 180° |
| 91c | 0° | | |
| 91c' | 180° | | |
| 91d | 90° | | |
| 91d' | 270° | | |
| 91e' | 180° | | |
| 91e' | 0° | | |

Die Funktionsweise des Systems läßt sich wie folgt darstellen: Die Partikel 30 werden entsprechend dem Pfeil in den Kanal eingeströmt. Wenn die Elektrodenreihen 91, 91a, 91b angesteuert sind, entsteht eine Feldbarriere, die die Partikel in den Zentralbereich der Strömung fokussiert. Über die Elektrodenreihen 91a, 91b werden die Partikel zueinander auf Abstand gebracht. Die Elektrodengruppen 91c-91f bilden einen Quadrupol, der seine Entsprechung 91c'-91f' auf der Oberseite des Kanals besitzt. Diese 8er-Gruppe von Elektroden fungiert entsprechend ihrer Ansteuerung als Feldkäfig und dient dem exakten Positionieren der Teilchen. Werden diese Elektrodengruppe oder zumindest die Elektrodensegmente 91d, 91f abgeschaltet, können die danach ausgeströmten Teilchen durch wahlweises Anschalten der Elektrodenreihen 91g oder 91h auf die rechte oder linke Seite des Kanals gelenkt werden. Es handelt sich bei diesem System somit um ein Partikel/Zell-Bewegungs- und Sortiermodul.

In Fig. 10 wird beispielhaft gezeigt, wie durch eine zu Fig. 9 verschiedene Ansteuerung der Elektrodensegmente eine neuartige Funktion des Systems erreicht werden kann. Eingeströmt wird diesmal eine Teilchengemisch 30a. 30b, 30c, bestehend aus verschieden großen und dielektrophoretisch unterschiedlich beeinflußbaren Teilchen (30a - dielektrophoretisch schwach zu beeinflussende Teilchen, 30b - Teilchen, größer als die Abstände zwischen den Elektroden und dielektrophoretisch gut ablenkbar, 30c - Teilchen, deutlich kleiner, als die Elektrodenpixelabstände gewählt wurden). Die Elektrodengruppen 101a, b fokussieren ausschließlich die großen Teilchen 30b auf eine Fangelektrode 102, wo sie festgehalten werden, während die Teilchen 30a und 30c nahezu unbeeinflußt den Kanal durchlaufen werden (Pfeil A). Wenn die Fangelektrode ab- und die Elektrodenpixelreihen 103 angeschaltet werden, bewegen sich die zurückgehaltenen Teilchen 30b nun entlang der eingezeichneten Bahnen und können separat abgefangen werden (Pfeil B). Die Kanalwände 104 können den Hauptkanal in mehrere Kanäle aufspalten.

Eine weitere Anwendung derartiger Arrays ist die universelle Anlage, d.h. potentielle Verwendbarkeit aller Elektrodensegmente oder -pixel, die jedoch in einem irreversiblen Prozeß bei der ersten Nutzung festgelegt bzw. aktiviert werden. Dies könnte z.B. durch Abschlagen einer Isolationsschicht, die Öffnung über einen elektrischen Impuls (Dauer rd. µs- bis s-Bereich, Spannung rd. 10 V bis einige 100 V), auf optischem Weg oder nach einem ähnlichen Prinzip erfolgen. Die dann freigelegte Struktur kann nur noch erweitert, nicht aber reduziert werden. Zumindest ist letzteres nur durch selektives Aufbringen neuer Isolationen möglich. Entsprechendes wäre über eine Oxidation denkbar. Ein bevorzugtes Mittel, im Kanal gegenüberliegende Elektrodenpixel von einer Isolationsschicht zu befreien, ist der dielektrische Durchschlag über Ansteuerung beider Elektroden mit kurzen elektrischen Impulsen.

Eine reversible Variante derartiger Aktivierungen von Punktelektroden kann über photoelektrische Effekte erreicht werden. Geeignete Halbleiter erlauben es, durch Belichtung in ihrer Leitfähigkeit deutlich verändert zu werden. Auf diesem Wege kann durch Belichtung über eine Maske auf einer oder beiden Seiten des Kanals das gewünschte Elektrodenmuster aktiviert werden.

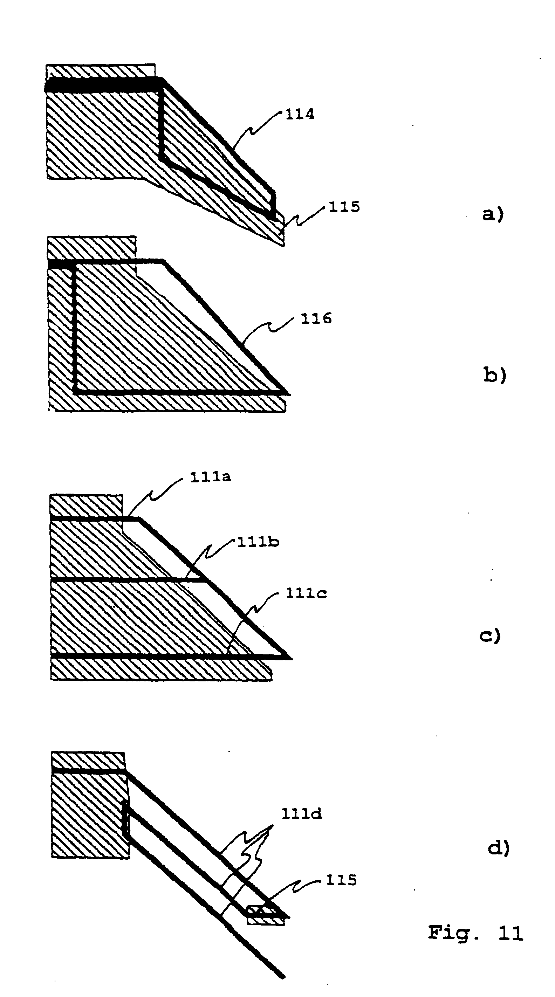

Ein weiteres wichtiges Kriterium zur Optimierung von Bandelektroden kann deren Umempfindlichkeit gegenüber einer Unterbrechung sein. Um trotzdem die Funktion aufrechtzuerhalten, sind Schleifen und Mehrfachspeisungen sinnvoll. Um die Verluste gering zu halten, können diese Teile der Elektroden mit einer Isolierschicht gegenüber der darüber befindlichen Suspension elektrisch getrennt werden. Einige beispielhafte Ausführungen sind in den Fig. 11a) bis d) zusammengestellt. 114 ist eine Ringelektrode mit sehr kleinem Loop und einer Isolationsschicht 115. Bei der Gestaltung b) handelt es sich um einen weiträumigeren Loop 116 mit ebenfalls teilweiser Isolation. Bei c) ist eine Mikroelektrodenmehrfacheinspeisung 111a bis 111c dargestellt. Die Einspeisungen können entweder permanent oder wahlweise nach Ausfall einer Zuführung angesteuert werden.

Bei der Gestaltung d) ist eine mehrfach gefaltete Bandelektrode 111d mit teilweiser Isolation 115 gezeigt. Wenn nicht gerade der vordere Teil (nahe der Einspeisung) ausfällt, wird die Funktion immer noch von einem der anderen Teile übernommen. Die dargestellten Elektrodentypen lassen sich in ihren Ausführungen auch sinngemäß kombinieren.

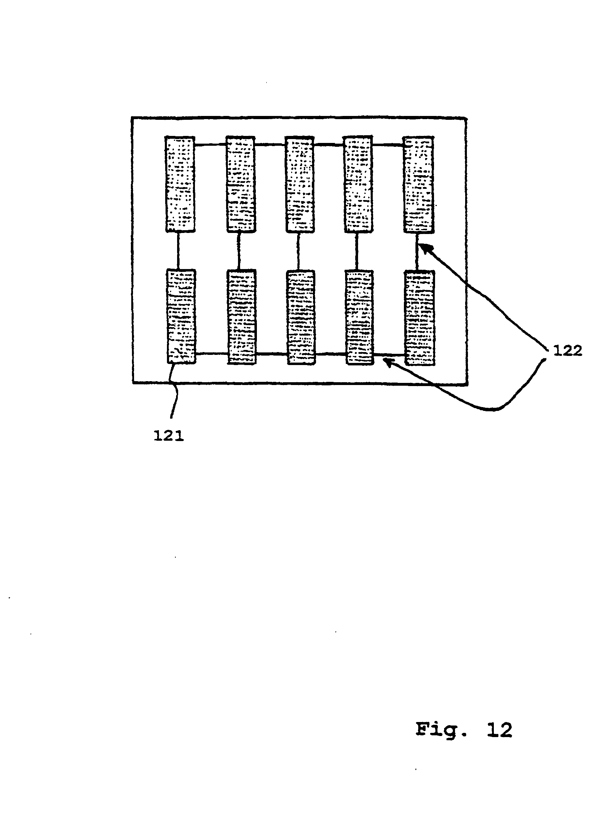

In Fig. 12 ist beispielhaft eine über elektrische Impulse programmierbare Elektrodenanordnung dargestellt. Die zehn rechtekkigen Elektroden 121 stehen untereinander über die Verbindungsstege 122 in elektrischem Kontakt. Diese Verbindungsstege können über einen Stromimpuls zwischen je zwei benachbarten Elektroden zerstört werden. Dies ermöglicht es, die Verschaltung zwischen den Elektroden per Stromimpuls festzulegen.

Es wird auch ein Verfahren zur Manipulation von Partikeln in fluidischen Mikrosystemen, insbesondere zur Bewegung von Partikeln in Mikrosystemen entlang vorbestimmter, zumindest abschnittsweise gerader Bahnen, und Vorrichtungen zur Implementierung eines derartigen Verfahrens, insbesondere ein fluidisches Mikrosystem, bei dem synthetische oder biologische Partikel in einer Suspensionsflüssigkeit manipuliert werden, und Anwendungen eines derartigen Mikrosystems beschrieben.

Fluidische Mikrosysteme mit flüssigkeitsdurchströmten Strukturen (z.B. Kanälen), in denen Mikroelektroden zur Beeinflussung von Partikeln (z.B. biologische Zellen) in den durchströmten Kanälen durch hochfrequente Felder auf der Basis negativer oder positiver Dielekrophorese angebracht sind, werden beispielsweise in der Publikation von G. Fuhr et al. in "Naturwissenschaften" (Bd. 81, 1994, S. 528 ff.) beschrieben.

Gewöhnlich werden fluidische Mikrosysteme von einer Flüssigkeit zum Vortrieb der Partikel durchströmt. Die auf beiden Kanallängsseiten (oben, unten) aufgebrachten Mikroelektroden führen zu einer Kompartimentierung des Kanals mittels hochfrequenter elektrischer Felder, mit denen die suspendierten Partikel in der gewünschten Weise, z.B. über Verzweigungen in Nachbarkanäle oder andere Strukturelemente, abgelenkt werden können. Schwierigkeiten bereiten vor allem die Einspülungen der Partikel jeweils an einem Kanalende und die Einstellung der in der Regel geringen Strömungsgeschwindigkeiten (einige µl/h), die mit steigender Miniaturisierung immer gravierende Einschränkungen mit sich bringen.

Ein genereller Nachteil herkömmlicher fluidischer Mikrosysteme besteht darin, daß zur gerichteten und einstellbaren Partikelbewegung eine Lösungsströmung erforderlich ist, deren Steuerung (z.B. der Strömungsgeschwindigkeit) Probleme bereitet.

Aus der Publikation von M. J. Madou et al. in "SPIE", Band 3259, 1998, S. 80 ff., ist ein Zentrifugal-Durchflußsystem bekannt, bei dem Flüssigkeitsströmungen in einem Mikrosystem nicht mit herkömmlichen Pumpen und Ventilen, sondern unter der Wirkung von Zentrifugalkräften eingestellt werden. Hierzu befindet sich das Mikrosystem in einem scheibenförmigen Träger in Gestalt einer CD-ROM-Scheibe. Analog zum Betrieb von CD-Speichermedien ist der Träger dazu vorgesehen, mit hoher Geschwindigkeit (im Bereich von 100 bis 10000 Umdrehungen pro Minute) gedreht zu werden. Die Flüssigkeiten im Mikrosystem bewegen sich unter der Wirkung der Zentrifugalkräfte radial nach außen. Simultan zu dieser Flüssigkeitsbewegung erfolgen im Mikrosystem bestimmte biochemische Reaktionen. Es ist auch vorgesehen, die Flüssigkeitsbewegung zum Teilchentransport, wie in einer herkömmlich gepumpten Flüssigkeitsströmung zu verwenden.

Die Zentrifugaltechnik nach M. J. Madou et al. besitzt die folgenden Nachteile. Sowohl die Erzielung einer genügenden Flüssigkeitsbewegung als auch eine möglichst behinderungsfreie Mitnahme von Partikeln mit der Flüssigkeit im scheibenförmigen, ebenen Rotor erfordern zwangsläufig die genannten hohen Drehzahlen des Trägers. Dadurch ergibt sich eine Einschränkung des herkömmlichen Zentrifugaldurchflußsystems auf bestimmte Grundfunktionen des herkömmlichen Zentrifugierens oder der Erzielung biochemischer Reaktionen. Die obengenannte Mikroelektrodentechnik zur Erzeugung hochfrequenter elektrischer Felder in den Mikrostrukturen ist nicht anwendbar. Ein weiterer Nachteil bezieht sich auf die mit der herkömmlichen Zentrifugaltechnik realisierten Partikelsortierungen und -zählungen. Diese sind nur möglich, indem Mikrokanäle mit einer Größe hergestellt werden, die der Größe der zu bearbeitenden Teilchen entspricht. Damit ist ein gegebenes Mikrosystem immer auf eine bestimmte Teilchengröße beschränkt. Außerdem kommt es bei der Handhabung von biologischen Partikeln (Zellen, Zellbestandteile) schnell zu Wechselwirkungen zwischen den Partikeln und der Kanalwand, die zu Kanalverstopfungen führen.

Es sind ferner Zentrifugensysteme allgemein bekannt, bei denen das Probenmaterial in der Zentrifuge nicht nur den Zentrifugalkräften, sondern auch zusätzlich z.B. magnetischen oder elektrischen Kräften ausgesetzt werden, um je nach dem Verhältnis der Zentrifugal- und der Zusatzkräfte spezifische Trenneffekte zu erzielen. Diese Zentrifugensysteme sind jedoch nicht zur Manipulierung biologischer Objekte verwendbar. Biologische Objekte (z.B. Zellen) werden nämlich in relativ stark leitfähigen Lösungen oder Suspensionen (Leitfähigkeiten im Bereich rd. 0.5 bis 3 Siemens/m) gehandhabt. Bei derartigen Leitfähigkeiten würde es in den herkömmlichen Zentrifugensystemen mit relativ großen Elektrodenflächen zu unerwünschten Aufheizungserscheinungen kommen. Die herkömmlichen Zentrifugensysteme sind daher auf Leitfähigkeiten von rd. 0.1 Siemens/m beschränkt.

Der hier beschriebene Aspekt ist darauf gerichtet, ein verbessertes Verfahren zur Manipulation von Partikeln in fluidischen Mikrosystemen anzugeben, mit dem die Nachteile herkömmlicher Mikrosysteme überwunden werden und das einen erweiterten Anwendungsbereich besitzt. Die Aufgabe ist es ferner, ein verbessertes fluidisches Mikrosystem mit einer gerichteten Partikelbewegung anzugeben, die vereinfacht und mit hoher Genauigkeit einstellbar ist. Die Aufgabe ist es auch, Anwendungen eines derart verbesserten Mikrosystems anzugeben.

Ein erster wichtiger Gesichtspunkt besteht darin, abweichend vom herkömmlichen Zentrifugaldurchflußsystem mit bewegten Flüssigkeiten zu einer Verfahrensweise überzugehen, bei der in einem fluidischen Mikrosystem unter der Wirkung von Zentrifugalkräften ausschließlich die zu manipulierenden Partikel bewegt werden, wobei im wesentlichen keine Flüssigkeitsströmungen oder -bewegungen im Mikrosystem auftreten. Hierzu werden eine Reihe von Maßnahmen realisiert, die insbesondere die Verwendung eines zumindest einseitig geschlossenen fluidischen Mikrosystems, die Anbringung eines solchen Mikrosystems an einer Schwingrotor-Zentrifugeneinrichtung und den Betrieb dieser Zentrifugeneinrichtung mit einer vorbestimmten Drehzahl umfassen, bei der sich die Partikel im Mikrosystem in gewünschter Weise bewegen.

Das Verfahren ermöglicht Zentrifugierungsvorgänge mit geringen Drehzahlen. Wegen der Verwendung eines Schwingrotorsystems, bei dem sich ein Rotor als Träger für das Mikrosystem von einer vertikalen Ausrichtung (bei Stillstand oder niedrigen Drehzahlen) zu einer horizontalen Ausrichtung (bei hohen Drehzahlen) aufrichtet, beeinflussen bei abnehmenden Drehzahlen zunehmend auch die Gravitationskraft die Bewegung der Partikel im Mikrosystem. Gemäß einem weiteren Gesichtspunkt wird auch eine Partikelbewegung in mindestens einseitig geschlossenen Mikrosystemen beschrieben, die sich im Stillstand mit vertikaler Ausrichtung des Mikrosystems befinden. Die Partikelbewegung erfolgt als Sedimentation unter Wirkung der Gravitationskraft.

Insbesondere werden derartige Mikrosysteme, die mit Mikroelektrodeneinrichtungen zur dielektrophoretischen Beeinflussung der Partikelbewegung ausgestattet sind, mit dem Prinzip des Zentrifugierens kombiniert. Die suspendierten Partikel bewegen sich aufgrund der Zentrifugalkräfte durch die Mikrokanäle oder andere Mikrostrukturen in einem Mikrosystem, in denen sie (ohne austreten zu können) unter Wirkung elektrischer Polarisationskräfte z.B. aufgetrennt, in eine vorher festgelegte Position gebracht, fusioniert, sortiert oder permeiert werden.

Ein wichtiger Vorteil besteht darin, daß erstmalig bei komplex strukturierten Mikrosystemen mit dielektrophoretischer Teilchenbeeinflussung auf den Einsatz von schwer steuerbaren und störanfälligen Pumpen oder Ventilen verzichtet werden kann, ohne daß eine Einschränkung der Funktionalität des Mikrosystems auftritt. Es bestehen keine Beschränkungen in Bezug auf die Kanalquerdimensionen. Es besteht die Möglichkeit, das Mikrosystem simultan mit der zugehörigen Steuerelektronik in Rotation zu versetzen. Wechselwirkungen von Partikeln (insbesondere biologischen Partikeln) mit Wandbereichen des Mikrosystems können ohne weiteres vermieden oder aber auch bei entsprechender Strukturierung zur Untersuchung von Bindungsvorgängen in vorbestimmter Weise erzielt werden.

Ein wichtiger Vorteil besteht darin, daß alle Partikel gleichermaßen der Zentrifugalkraft ausgesetzt werden und sich entsprechend einer Bezugsrichtung entlang vorbestimmer Kanäle bewegen und die Trennung z. B. in verschiedene Teilkanäle oder Reservoire ausschließlich über Ablenkkräfte erzielt wird, die unabhängig von der Zentrifugalkraft partikelspezifisch wirken. Die Ablenkkräfte besitzen eine von der Bezugsrichtung abweichende Richtung, wobei der Winkelunterschied vorzugsweise kleiner als 90° ist. Über die Zentrifugalkraft wird lediglich die Partikelgeschwindigkeit eingestellt. Nach der Trennung können die Zusatzkräfte abgeschaltet werden, ohne das sich die Partikel wieder vermengen. Es ist ein unerwartetes und wichtiges Merkmal, daß durch den Einsatz einer Schwingrotorzentrifuge der Kontakt von Partikeln mit Probenkammerwandungen vermieden werden kann, was besonders bei biologischen Objekten von Bedeutung ist.

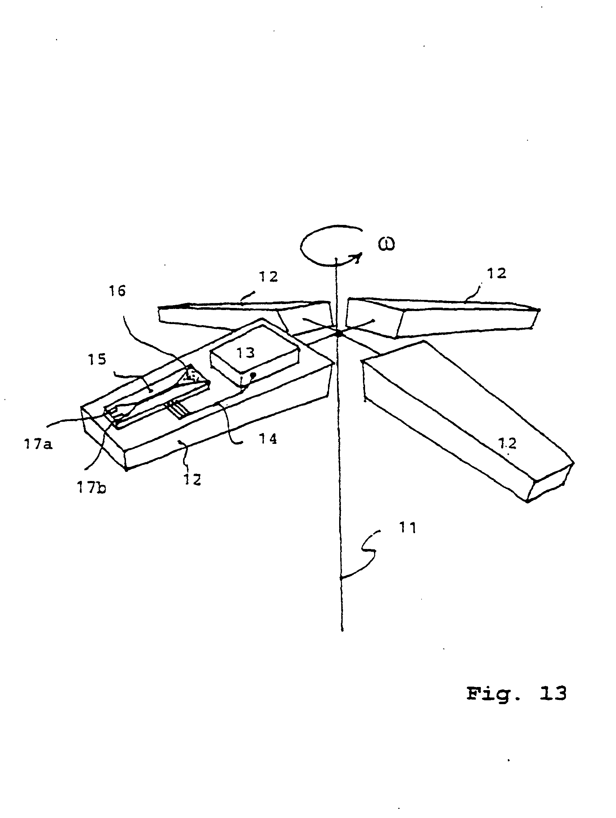

Die Figuren 13 bis 15 zeigen eine schematische Perspektivansicht eines Aufbaus eines Zentrifuge mit einem Mikrosystem, eine schematische Draufsicht auf ein Mikrosystem, das zur Teilchentrennung eingerichtet ist, und eine schematische Draufsicht auf ein programmierbares Beladungsmikrosystem.

Die hier beschriebenen Beispiele beziehen sich auf die Kombination eines Mikrosystems, das mit einer Mikroelektrodeneinrichtung zur Ausübung negativer oder positiver Dielektrophorese ausgestattet ist (dielektrophoretisches Mikrosystem), mit einer Schwingrotorzentrifugeneinrichtung. Sowohl das dielektrophoretische Mikrosystem (abgesehen von der mindestens einseitigen Verschließbarkeit von Kanalstrukturen) als auch die Schwingrotorzentrifugeneinrichtung sind jeweils an sich bekannt, so daß auf deren technische Einzelheiten hier nicht weiter eingegangen wird. Es wird betont, daß der Begriff der Schwingrotorzentrifugeneinrichtung hier auch im weitesten Sinne dahingehend zu verstehen ist, daß jede Zentrifugeneinrichtung mit mindestens einem drehzahlabhängig aufrichtbaren Rotor eingeschlossen ist, der selbst das Mikrosystem und die zugehörige Steuerung bildet, in den das Mikrosystem und die zugehörige Steuerung integriert oder auf den das Mikrosystem und die zugehörige Steuerung aufgesetzt sind.

Die manipulierten Partikel können synthetische Teilchen oder biologische Objekte umfassen. Die synthetischen Teilchen sind beispielsweise membranumhüllte Gebilde, wie Liposomen oder Vesikeln, oder sogenannte Beads oder auch Makromoleküle. Die biologischen Objekte umfassen beispielsweise biologische Zellen oder Bestandteile von diesen (z.B. Zellorganellen), Bakterien oder Viren. Die Partikel können auch Aggregate oder Zusammenballungen derartiger Teilchen und/oder Objekte sein. Vorzugsweise wird mit zellphysiologisch oder medizinisch relevanten Fluiden mit Leitfähigkeiten unterhalb 5 Siemens/m implementiert.

Fig. 13 ist eine schematische Übersichtsdarstellung einer Vorrichtung zur Illustration der Anbringung eines dielektrophoretischen Systems an einer Zentrifugeneinrichtung.

An einem üblichen oder anwendungsabhängig modifizierten Rotor einer Zentrifuge mit der Drehachse 11 befinden sich vier Aufnahmen 12, in die jeweils paßgerecht und für die applizierten Drehzahlen entsprechend ein Mikrosystem 15 und eine Steuerelektronik 13 zur Ansteuerung des Mikrosystems mit hochfrequenten Wechselsignalen verschiedener Phasenlage und Amplitude eingesetzt sind. Die Steuerelektronik ist über Kabel 14, Stecker oder anderweitig mit dem Mikrosystem 15 verbunden. Die Energieversorgung der Steuereinrichtung erfolgt vorzugsweise über eine elektrische Verbindung, (umlaufender Kontakt) mit dem festen Laborsystem. Das Mikrosystem hat ein Eingangsdepot 16, das anwendungsabhängig verschieden groß ausgelegt sein kann und vor der Zentrifugation mit einer Teilchen- oder Zellsuspension gefüllt wird. Vom Eingangsdepot 16 aus verläuft eine Kanalstruktur, deren Einzelheiten weiter unten erläutert werden, bis zu Auffangzonen 17a, 17b, die ein zumindest während des Zentrifugierens geschlossenes Ende des Mikrosystems 15 bilden. Dies bedeutet, daß das Ende des Mikrosystems entweder dauerhaft abgeschlossen oder bei Stillstand der Vorrichtung durch entsprechende Verbindungselemente geöffnet und an vorbestimmte Zusatzsysteme zur Probenübertragung angeschlossen werden kann. Das Mikrosystem 15 ist so auf der Aufnahme 12 angeordnet, daß bei Betrieb der Zentrifugeneinrichtung (Drehung des Rotors um die Drehachse 11 mit der Drehfrequenz ω) die auf das Mikrosystem 15 und in diesem befindliche Partikel wirkenden Zentrifugalkräfte in der Bezugsrichtung vom Eingangsdepot 18 hin zu den Auffangzonen 17a, 17b gerichtet sind. Die Aufnahmen 12 sind verschwenkbar am Rotor (nicht dargestellt) angebracht. Beim Stillstand der Zentrifuge sind die Aufnahme 12 im wesentlichen vertikal oder mit einem geringen Winkel gegenüber der Drehachse ausgerichtet. Beim Zentrifugenbetrieb richten sich die Aufnahmen 12 drebzahlabhängig in einen größeren Winkel bis hin in die horizontale Ausrichtung senkrecht zur Drehachse 11 auf. Unter der Wirkung der Gravitationskraft (bei Stillstand der Zentrifuge) bwz. der Zentrifugalkräfte durchlaufen die Teilchen das elektronisch gesteuerte Mikrokanalsystem und sammeln sich in den Auffangzonen (z.B. am geschlossenen Ende des von der Rotorachse wegweisenden Teils des Mikrosystems).

Bei diesem Durchlauf werden die Partikel nach vorbestimmten Programmen (s. unten) behandelt. Da die Teilchen in Abhängigkeit von ihrer Dichte verschiedene Bewegungen ausführen und Endpositionen einnehmen, wird der Vorteil der Zentrifugaltrennung und -bewegung mit den Möglichkeiten der programmierbaren Dielektrophorese kombiniert. In der Regel wird negative Dielektrophorese, in Ausnahmefällen auch positive Dielektrophorese der Teilchen genutzt. Ein weiterer Vorteil ist die Steuerung der Teilchenbewegung über die Rotationsgeschwindigkeit (ω) des Rotors 11. Da hierbei ebenfalls programmierbare Variationen durchlaufen werden können, ist ein zweiter Komplex von festlegbaren Parametern bei der Partikelmanipulation gegeben.

Die Zentrifugeneinrichtung ist mit einer (nicht dargestellten) Drehzahlsteuerung versehen, die für eine reproduzierbare und genaue Drehzahleinstellung insbesondere in niedrigen Drehzahlbereichen eingerichtet ist. Die Drehzahl wird anwendungsabhängig je nach der gewünschten Geschwindigkeit der zu manipulierenden Teilchen und in Abhängigkeit vom konkreten Zentrifugenaufbau gewählt. Die interessierenden Partikelgeschwindigkeiten liegen für biologische Partikel (z.B. Zellen) unterhalb von rd. 500 µm/s (vorzugsweise im Bereich von 50 bis 100 µm/s) und für synthetische Partikel (z.B. Latex-Beads) bei höheren Geschwindigkeiten (z.B. einige mm/s). Die Drehzahl der Zentrifugeneinrichtung wird entsprechend den Zusammenhängen von Drehzahl und Zentrifugalkraft in Abhängigkeit von der Größe bzw. Massendichte der Partikel gewählt. Die folgenden Angaben beziehen sich auf einen Abstand des Mikrosystems von der Rotorachse im Bereich von 1 bis 10 cm. Für Partikeldurchmesser im Bereich von 50 bis 600 nm (z.B. Viren) können die Drehzahlen beispielsweise im Bereich von 1 bis 1000 U/min liegen. Bei Partikeln mit einem Durchmesser von rd. 5 µm werden Drehzahlen bis zu 100 U/min bevorzugt, wobei jedoch auch höhere Drehzahlen einstellbar sind. Bei besonders kleinen Partikeln, z.B. Makromoleküle sind auch noch höhere Drehzahlen realisierbar. Für biologische Zellen ergeben sich bei einem Abstand des Mikrosystems von rd. 5 bis 10 cm von der Drehachse 11 Drehzahlen im Bereich von wenigen Umdrehungen pro Minute bis zu einigen 100 (z. B. 600) Umdrehungen pro Minute, vorzugsweise unterhalb 100 U/min. Die erzielbaren Zentrifugalkräfte liegen im Bereich von pN bis nN. Die Zentrifugeneinrichtung ist jedoch auch für größere Drehzahlen ausgelegt, die insbesondere für kleine Partikel oder für Reinigungsoder Spülzwecke eingestellt werden können. Diese erhöhten Drehzahlen können bis zum Bereich der Drehzahlen herkömmlicher Laborzentrifugen reichen.

Die Drehzahl der Zentrifuge wird auch in Abhängigkeit von den dielektrophoretischen Kräften ausgewählt, die auf die Partikel im Mikrosystem wirken. Die dielektrophoretischen Kräfte sind als Polarisationskräfte von der Teilchenart und -größe abhängig. Die Drehzahl wird vorzugsweise so ausgewählt, daß die Zentrifugalkräfte auf die Partikel kleiner oder gleich den dielektrophoretischen Kräften sind. Falls diese nicht bekannt sind, kann die Drehzahl auch in Bezug auf das folgende Kriterium ausgewählt werden. Die Teilchen müssen sich so langsam durch die Kanalstruktur bewegen, daß beim Vorbeitritt an den Mikroelektrodeneinrichtungen genügend Zeit zur dielektrophoretischen Ablenkung bleibt. Die Wirksamkeit oder Unwirksamkeit der dielektrophoretischen Ablenkung in Abhängigkeit von der Drehzahl kann mit geeigneten Sensoren optisch oder elektrisch erfaßt werden.

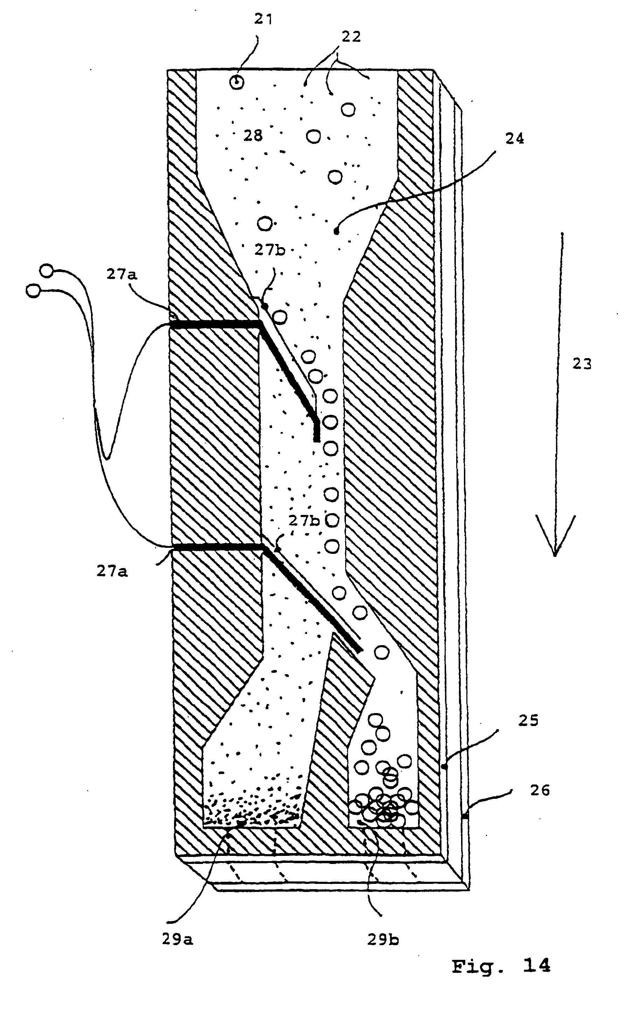

Fig. 14 zeigt in schematischer Weise ein Mikrosystem zur Auftrennung eines Partikelgemisches, bestehend aus größeren Teilchen 21 (z.B. Zellen) und kleinen Teilchen 22, die in einer Suspension vorliegen. Die Zentrifugalkräfte wirken in Pfeilrichtung 23 (Bezugsrichtung). Die typischen Abmessungen der Kanalstruktur 24 sind die folgenden:
- Breite:: einige 10 µm bis zu einigen mm (typischerweise: 200 - 400 µm)
- Länge:: einige mm bis zu einigen cm (typischerweise: 20 - 50 mm)
- Höhe:: einige µm bis zu einigen 100 µm (typischerweise: 50 µm)

Auf der Oberseite 25 und Unterseite 26 des Kanals 24 sind Mikroelektroden 27a, 27b gegenüberliegend angeordnet, die bei Ansteuerung mit einer Wechselspannung (in der Regel einer Frequenz im MHz-Bereich und einer Amplitude von einigen Volt) quer zum Kanal Feldbarrieren erzeugen, die über negative (bedingt auch positive) Dielektrophorese die Teilchen ablenken (im hier gezeigten Fall die großen Teilchen).

Die Kanalstruktur 24 reicht vom Eingangsdepot 28 zu den geschlossenen Kanalenden 29a, 29b, in die sich der in einem mittleren Abschnitt gerade Kanal verzweigt. Ein erstes Paar der Mikroelektroden 27a, 27b ist unmittelbar am kanalseitigen Ende des Eingangsdepots 28 zur Ausbildung einer Feldbarriere angeordnet, die schräg in den Kanal hineinragt und die Aufgabe besitzt, die großen Teilchen 21 in den in Draufsicht rechten Teil des Kanals 24 zu drängen. Ein zweites Paar der Mikroelektroden 27a, 27b ist unmittelbar vor der Verzweigung zu den Kanalenden 29a, 29b angeordnet und bildet eine Feldbarriere, die schräg über die Kanalbreite bis in die zum Kanalende 29b führende Abzweigung reicht und dazu vorgesehen ist, die großen Teilchen 21 zu diesem Kanalende hin zu führen.

Ein Manipulationsverfahren, das bei diesem Beispiel auf eine Trennung der Teilchen gerichtet ist, umfaßt die folgenden Schritte.

Vor der Zentrifugation wird das Mikrosystem mit einer geeigneten Flüssigkeit gefüllt. Dabei ist das Mikrosystem bereits in eine Aufnahme 12 der Zentrifuge (s. Fig. 13) eingebaut. Der Einbau kann aber auch nach der Befüllung des Mikrosystems erfolgen. Kurz vor Beginn der Zentrifugation werden die Elektroden 27a, 27b angesteuert und im Eingangsdepot 28 wird z.B. mit einer Pipettiereinrichtung die Suspension der zu trennenden Teilchen zugegeben. Die Zentrifugeneinrichtung ist zunächst noch im Ruhezustand, d.h. das Mikrosystem ist vertikal oder zur Vertikalen leicht geneigt ausgerichtet. Die Gravitationskraft, die auf die Teilchen wirkt, führt zu einem masseabhängig verschieden schnellen Absinken in die Kanalstruktur (Sedimentation). Die weitere Bewegung der Teilchen hin zu den Kanalenden erfolgt je nach der gewünschten Teilchengeschwindigkeit ausschließlich unter der Wirkung der Gravitationskraft oder unter der gemeinsamen Wirkung der Gravitationskraft und der Zentrifugalkräfte. Die Zentrifugation kann somit als Sedimentation unter der Wirkung einer künstlich erhöhten Fallbeschleunigung aufgefaßt werden. Die sich bewegenden Teilchen werden durch das elektrische Feld des ersten Paares der Mikroelektroden größenabhängig getrennt.

Die Darstellung in Fig. 14 zeigt die Verhältnisse während der Sedimentation bzw. Zentrifugation. Durch die exakt einstellbaren Zentrifugalkräfte über die Rotationsgeschwindigkeit bewegen sich die Teilchen in den unteren Teil des Mikrosystems. Entsprechend der üblichen Zentrifugationsprinzipien sedimentieren die Teilchen mit der größten Dichte zuerst. Da die Teilchen 21 durch die elektrische Feldbarriere im Kanal nach rechts verschoben werden, während die Teilchen 22 davon unbeeinflußt bleiben, so ergibt sich in den Kanalenden 29a, 29b eine Trennung beider Teilchenarten. Die Teilchen in jedem der Kanalenden ordnen sich zusätzlich wie bei der üblichen Zentrifugation entsprechend ihrer Dichte an. Das dargestellte Mikrosystem kann als Grundform einer Vorrichtung betrachtet werden, wobei diese Grundform anwendungsabhängig vergrößert, erweitert oder mit weiteren Mikrostrukturen kombiniert werden kann. Der Vorteil besteht darin, daß keine Lösungsströmung entsteht und dennoch die Partikelbewegung gerichtet und einstellbar ist. Derartige Systeme können auch entgegengesetzte Bewegungen erzeugen, wenn die Teilchen einen Auftrieb besitzen.

Ausgehend von der dargestellten Grundform kann ein Mikrosystem beliebig erweitert werden, wie es an sich von den dielektrophoretischen Mikrosystemen bekannt ist. Demnach kann die Kanalstruktur insbesondere mehrere, über Verzweigungen miteinander verbundene Einzelkanäle aufweisen. Die Kanäle können gerade oder gekrümmt sein. Gekrümmte Kanalformen (z.B. Bögen, Mäander, Biegungen, Winkel usw.) können insbesondere zur Untersuchung von Bindungsunterschieden von Partikeln mit den Kanalwänden verwendet werden.

Gemäß einer weiteren Modifikation kann das Mikrosystem an der Aufnahme 12 (s. Fig. 13) drehbar angebracht sein. Während eines ersten Zentrifugationsvorganges erfolgt in einer ersten Mikrosystemorientierung z.B. eine Teilchentrennung gemäß Fig. 2. Anschließend wird die Orientierung des Mikrosystems um 180° verändert, so daß die Gravitations- und/oder Zentrifugalkräfte entgegengesetzt der Pfeilrichtung 23 wirken. Die Kanalenden 29a, 29b übernehmen dann die Funktion von Eingangsdepots, von denen bei Vorhandensein geeigneter Kanalstrukturen (zusätzliche seitliche Abzweigungen) eine weitere Verteilung der getrennten Teilchen in Untergruppen oder eine bestimmte Behandlung (Beladen mit Stoffen, Elektroporation u. dgl.) erfolgen kann. Es sind auch in Abhängigkeit von der Kanalstruktur andere Orientierungsänderungen als die genannte 180°-Umkehr möglich. Es besteht ferner die Möglichkeit, die Aufnahme 12 so zu gestalten, daß das Mikrosystem während der Zentrifugation gedreht wird.

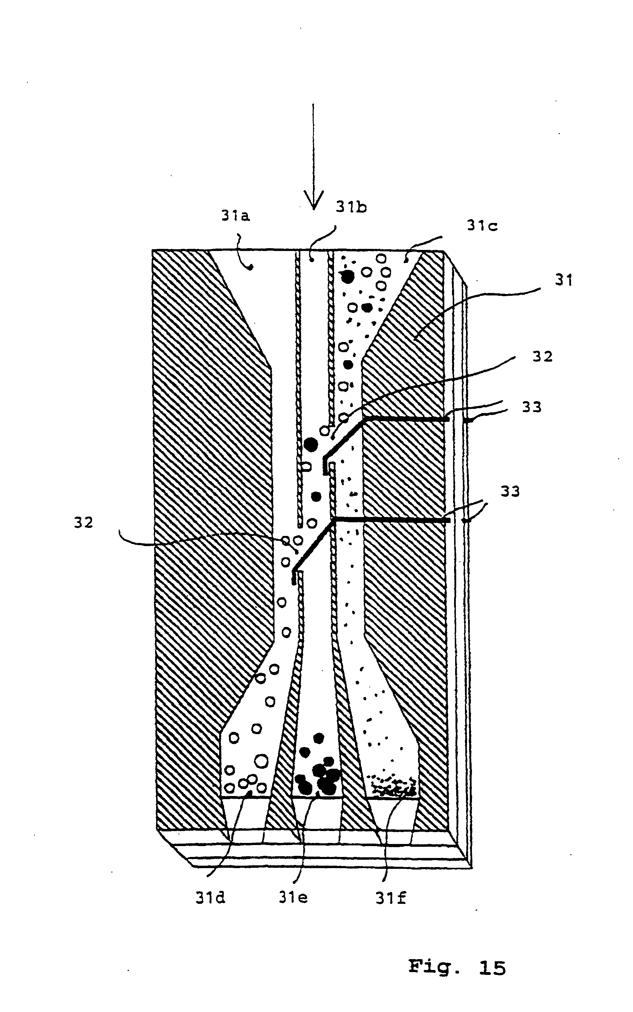

Eine weitere Ausführungsform, nämlich ein programmierbares Beladungsmikrosystem für Zellen oder Teilchen ist in Fig. 15 gezeigt. Hier ist der Zentrifugationskanal in drei Teile 31a, 31b, 31c unterteilt. In den Zwischenwänden befinden sich Öffnungen 32, durch die wieder Elektroden 33 auf der Ober- und Unterseite des Kanals hindurchreichen. Die Öffnungen sind der Teilchengröße angepaßt (typischerweise 5- bis 20-fach größer als der Durchmesser). Zu Beginn werden in jeden der Kanalteile 31a bis 31c verschiedene Lösungen eingefüllt, die der chemischen Veränderung oder Beladung der Partikel dienen. Danach werden in einen Kanalteil (hier z.B. 31c) die Teilchen eingefügt. Durch die Zentrifugation gelangen die Teilchen (z.B. zuerst die schwarzen, dann die hellen) an die Elektroden 33 und können so automatisch über die elektrischen Feldbarrieren durch die Öffnungen 32 in die Nachbarlösungen überführt werden.

Auch hier kommt es zu einer Sortierung in den drei Kanalenden 31d, 31e, 31f und gleichzeitig zu einer Anordnung der Teilchen entsprechend der Masseunterschiede.

Weitere Eigenschaften der Mikrosysteme bestehen darin, daß sie Öffnungen (Zuflüsse, Durchflüsse, Abflüsse) besitzen können, die sich verschließen lassen, so daß die Teilchen nach der Zentrifugation oder davor leicht entnommen oder eingefügt werden können. Ferner können all die Mikroelektrodenelemente (Halteelektroden für Teilchen, Mikrofeldkäfige etc.) eingebaut werden, die für die dielektrophoretische Beeinflussung von Teilchen an sich bekannt sind und bei herkömmlichen Mikrosystemen, die mit strömenden Flüssigkeiten arbeiten, eingesetzt werden. Aufgrund des Zusammenwirkens der Gravitations- bzw. Zentrifugalkräfte mit den dielekrophoretischen Kräften ist das Verfahren eine elektrisch gesteuerte oder aktive Zentrifugation. Zusätzlich können Kombinationen mit der Einwirkung optischer Kräfte (Laser-Tweezer), magnetischer Kräfte (Einwirkung auf magnetische Partikel) oder mechanischer Kräfte in Form von Ultraschallkräften vorgesehen sein.

Anwendungsgebiete sind insbesondere:
Zelltrennung/-fraktionierung, Zellsortierung, Zellbeladung (molekular, Nanoteilchen, Beads), Zellentladung (molekular), Zellpermeation (sog. Elektroporation), Zellfusion (sog. Elektrofusion), Zellpärchenbildung, und Zellaggregatbildung.

Das Verfahren ist nicht auf bestimmte Lösungs- oder Suspensionsflüssigkeiten beschränkt. Es ist vorteilhaft, wenn die Viskosität der im Mikrosystem enthaltenen Flüssigkeit bekannt ist. Bei bekannter Viskosität läßt sich die Drehzahl zur Einstellung einer bestimmten Partikelgeschwindigkeit auf der Grundlage von Tabellenwerten oder durch einen Programmalgorithmus ermitteln. Alternativ ist es jedoch auch möglich, die tatsächliche Geschwindigkeit der Partikel im Mikrosystem während der Zentrifugation zu erfassen (z.B. mit einem optischen Sensor) und die Drehzahl zur Einstellung einer bestimmten Partikelgeschwindigkeit zu regeln. Es kann vorgesehen sein, daß in verschiedenen Teilbereichen des Kanalstrukturen, z.B. in parallel verlaufenen Kanälen, die nur über eine Öffnung miteinander verbunden sind, Flüssigkeiten mit verschiedenen Viskositäten enthalten sind. In diesem Fall werden jedoch Viskositäten bevorzugt, bei denen sichergestellt ist, daß die Diffusion der Flüssigkeiten durch die Öffnung über den Zentrifugat-ionszeitraum verhältnismäßig klein oder vernachlässigbar klein ist.

Falls die Massendichte der Partikel kleiner als die Flüssigkeit im Mikrosystem ist, kann das Verfahren entsprechend abgewandelt implementiert werden, indem Partikel gegebenenfalls auf der der Drehachse abgewandten Seite des Mikrosystems eingebracht werden und unter Wirkung des Auftriebs oder unter kombinierter Wirkung des Auftriebs und der Zentrifugalkräfte zum anderen Ende des Mikrosystems wandern.

Das Mikrosystem wird anwendungsabhängig in Bezug auf die Kanalstruktur und die Ausrichtung der Elektrodeneinrichtungen angepaßt. Die Kanalquerdimensionen sind in der Regel wesentlich größer als die Durchmesser der einzelnen Partikel. Dadurch wird vorteilhafterweise ein Verstopfen der Kanäle vermieden. Sind lediglich Partikel mit besonders geringen Dimensionen zu manipulieren (z.B. Bakterien oder Viren oder Zellorganellen), so können die Kanaldimensionen entsprechend verringert werden, z.B. auf Beträge unterhalb 10 µm.

Das Verfahren wird mit einem Mikrosystem implementiert, das mindestens einseitig geschlossen ist. Das geschlossene Ende kann ein geschlossenes Kanalende, eine geschlossene Sammelzone oder auch ein geschlossener Hohlraum im Mikrosystem sein. Bei der Partikelmanipulation erfolgt im wesentlichen keine Flüssigkeitsbewegung hin zu dem geschlossenen Ende. Dies bedeutet, insbesondere bei Realisierung von Sammelzonen oder Hohlräumen am geschlossenen Ende, daß diese wie das gesamte Mikrosystem zu Beginn der Partikelmanipulation mit der Lösung oder Suspension für die Teilchen gefüllt ist.

Falls es beim Manipulieren der Partikel zu Zusammenballungen oder vorübergehenden Verstopfungen der Kanalstrukturen kommt, so ist vorgesehen, die Drehzahl der Zentrifuge kurzzeitig zu erhöhen, um so die zusammenhaftenden Partikel abzulösen und weiter zu bewegen.

## Patentansprüche

1. Mikrosystem, das zur dielektrophoretischen Manipulation von Partikeln (30) in einer Suspensionsflüssigkeit in einem Kanal (21) eingerichtet ist und eine Elektrodenanordnung (10) mit mindestens einer Elektrode (11, 11a, 11b, 12, 13) enthält, die an einer seitlichen Wand des Kanals (21) angeordnet ist,
**dadurch gekennzeichnet, dass**
die Elektrode (11, 11a, 11b, 12, 13) aus mindestens einer Metallschicht besteht, auf der eine strukturierte Isolationsschicht (15, 45, 62, 115) mit vorbestimmten Ausnehmungen gebildet ist, in denen die Metallschicht mit Elektrodenflächen zum Kanal hin freiliegt, so dass durch die freiliegenden Elektrodenflächen eine Vielzahl von Elektrodensegmenten (41, 51, 61 a-d, 71, 81, 111 a-d, 114, 116) gebildet werden, zwischen denen auf der zum Kanal weisenden Oberfläche des Mikrosystems die Isolationsschicht (15, 45, 62, 115) vorgesehen ist, wobei die Elektrodensegmente zur Erzeugung mindestens eines Feldgradienten zur Beeinflussung der Bewegungsbahnen der Partikel (30) im Kanal (21) eingerichtet sind.

2. Mikrosystem gemäß Anspruch 1, bei der die Elektrodensegmente (41, 51, 71) einer Elektrode elektrisch miteinander verbunden sind.

3. Mikrosystem gemäß Anspruch 1 oder 2, bei der die Elektrodensegmente punkt- oder strichförmig zur Erzeugung von quadratischen oder bandförmigen Elektrodensegmenten ausgebildet sind.

4. Mikrosystem gemäß Anspruch 1, bei der die Elektrodensegmente (81) voneinander elektrisch getrennt und einzeln ansteuerbar sind.

5. Mikrosystem gemäß Anspruch 4, bei der die Elektrodensegmente (81, 91, 101) matrixartig als Elektrodenarray angeordnet sind.

6. Mikrosystem gemäß einem der vorhergehenden Ansprüche, bei der die Elektrodensegmente in Form gerader oder gekrümmter Reihen angeordnet sind, die sich jeweils auf einer Kanalwand vom Rand des Kanals hin zu dessen Mitte zur Bildung einer trichterförmigen Feldbarriere erstrecken.

7. Verwendung eines Mikrosystems gemäß einem der Ansprüche 1 bis 6 zur Manipulation synthetischer oder biologischer Partikel in Mikrosystemen auf der Basis von negativer oder positiver Dielektrophorese.

## Claims

1. A microsystem arranged for dielectrophoretic manipulation of particles (30) in a suspending liquid in a channel (21), including an electrode arrangement with at least one electrode (11, 11a, 11b, 12, 13) which is arranged on a sidewall of the channel (21), **characterized in that** the electrode (11, 11a, 11b, 12, 13) consists of at least one metal layer on which there is formed a structured insulating layer (15, 45, 62, 115) with predetermined openings in which the metal layer electrode surfaces exposed to the channel, so that a plurality of electrode segments (41, 51, 61 a-d, 71, 81, 111 a-d, 114, 116) are formed by the exposed electrode surfaces, between which the insulating layer (15, 45, 62, 115) is provided on the surface of the microsystem facing the channel, wherein the electrode segments are arranged for generation of at least one field gradient for affecting the paths of movement of the particles (30) in the channel (21).

2. A microsystem according to claim 1, in which the electrode segments (41, 51, 71) of an electrode are connected electrically together.

3. A microsystem according to claim 1 or 2, in which the electrode segments are of point or stroke form for generating square or strip form electrode segments.

4. A microsystem according to claim 1, in which the electrode segments (81) are electrically separate from one another and can be controlled individually.

5. A microsystem according to claim 4, in which the electrode segments (81, 91, 101) are arranged like a matrix as an electrode array.

6. A microsystem according to any of the preceding claims, in which the electrode segments are arranged in the form of straight or curved rows, which each extend on a channel wall from the edge of the channel towards its middle to form a funnel-shaped field barrier.

7. Use of a microsystem according to any of claims 1 to 6 for manipulation of synthetic or biological particles in microsystems on the basis of negative or positive dielectrophoresis.

## Revendications

1. Microsystème qui est agencé pour la manipulation diélectrophorétique de particules (30) dans un liquide en suspension dans un conduit (21) et qui comprend un dispositif à électrodes (10) avec au moins une électrode (11, 11a; 11b, 12, 13) qui est installée dans une paroi latérale du conduit (21), **caractérisé en ce que** l'électrode (11, 11a; 11b, 12, 13) est constituée d'au moins une couche de métal sur laquelle est formée une couche d'isolation structurée (15, 45, 62, 115) avec des creux prédéterminés dans lesquels la couche de métal avec des surfaces d'électrodes est à découvert en direction du conduit, de sorte qu'une pluralité de segments d'électrodes (41, 51, 61 a-d, 71, 81, 111 a-d, 114, 116), entre lesquels la couche d'isolation (15, 45, 62, 115) est prévue sur la surface du microsystème dirigée vers le conduit, est formée par les surfaces d'électrodes à découvert, moyennant quoi les segments d'électrodes sont agencés pour créer au moins un gradient de champ pour influencer les trajectoires de déplacement des particules (30) dans le conduit (21).

2. Microsystème selon la revendication 1, dans lequel les segments d'électrodes (41, 51, 71) d'une électrode sont raccordés électriquement les uns aux autres.

3. Microsystème selon la revendication 1 ou 2, dans lequel les segments d'électrodes sont conçus en forme de points ou de traits pour créer des segments d'électrodes carrés ou en forme de bande.

4. Microsystème selon la revendication 1, dans lequel les segments d'électrodes (81) sont séparés électriquement les uns des autres et peuvent être commandés individuellement.

5. Microsystème selon la revendication 4, dans lequel les segments d'électrodes (81, 91, 101) sont disposés à la manière d'une matrice comme un réseau d'électrodes.

6. Microsystème selon l'une quelconque des revendications précédentes, dans lequel les segments d'électrodes sont disposés sous forme de rangées droites ou courbes qui s'étendent respectivement sur une paroi du conduit entre le bord du conduit jusqu'à son centre pour former une barrière de champ en forme d'entonnoir.

7. Utilisation d'un microsystème selon l'une quelconque des revendications 1 à 6 pour la manipulation de particules synthétiques ou biologiques dans des microsystèmes sur la base d'une diélectrophorèse négative ou positive.
